# EUROPÄISCHE PATENTSCHRIFT

(11) **EP 3 080 309 B1**
(45) Veröffentlichungstag und Bekanntmachung des Hinweises auf die Patenterteilung: **07.06.2023**
(21) Anmeldenummer: 14815582.3
(22) Anmeldetag: 11.12.2014
(51) Int. Cl.: C21B 5/06, C21C 5/38, C21B 7/00, C25B 1/04, C07C 29/15

(54) **ANLAGENVERBUND ZUR STAHLERZEUGUNG UND VERFAHREN ZUM BETREIBEN DES ANLAGENVERBUNDES**
PLANT COMBINATION FOR PRODUCING STEEL AND METHOD FOR OPERATING THE PLANT COMBINATION
ENSEMBLE D'INSTALLATIONS PERMETTANT LA PRODUCTION D'ACIER ET PROCÉDÉ PERMETTANT DE FAIRE FONCTIONNER L'ENSEMBLE D'INSTALLATIONS

(30) Priorität: 12.12.2013 DE 102013113913
(43) Veröffentlichungstag der Anmeldung: 19.10.2016
(60) Teilanmeldung: 19193959.4
(73) Patentinhaber: thyssenkrupp AG, 45143 Essen (DE)
(72) Erfinder: ACHATZ, Reinhold, 45259 Essen (DE); WAGNER, Jens, 60439 Frankfurt a.M. (DE); OLES, Markus, 45527 Hattingen (DE); SCHMÖLE, Peter, 44141 Dortmund (DE); KLEINSCHMIDT, Ralph, 45472 Mülheim a.d.R. (DE); KOLBE, Bärbel, 58452 Witten (DE); KRÜGER, Matthias Patrick, 44625 Herne (DE); MEISSNER, Christoph, 44135 Dortmund (DE)
(74) Vertreter: thyssenkrupp Intellectual Property GmbH
(86) Internationale Anmeldenummer: PCT/EP2014/003320
(87) Internationale Veröffentlichungsnummer: WO 2015/086154

(56) Entgegenhaltungen:
- EP-A1- 0 244 551
- EP-A1- 2 816 096
- EP-A2- 0 200 880
- WO-A1-2014/202557
- US-A1- 2006 027 043
- SCHMOELE P ET AL: "ECOLOGICAL HOT METAL PRODUCTION USING COKE PLANT AND BLAST FURNACE ROUTE//PRODUCTION ECOLOGIQUE DE FONTE PAR LA FILIERE COKERIE ET HAUT-FOURNEAU", REVUE DE METALLURGIE - CAHIERS D'INFORMATIONS TECHNIQUES, REVUE DE METALLURGIE. PARIS, FR, Bd. 102, Nr. 3, 1. März 2005 (2005-03-01), Seiten 171-182, XP001230940, ISSN: 0035-1563, DOI: 10.1051/METAL:2005140
- HAMID GHANBARI ET AL: "Optimal design and operation of a steel plant integrated with a polygeneration system", AI CH E JOURNAL, vol. 59, no. 10, 1 October 2013 (2013-10-01), pages 3659-3670, XP055501257, US ISSN: 0001-1541, DOI: 10.1002/aic.14098

## Beschreibung

Die Erfindung betrifft ein Verfahren zum Betreiben des Anlagenverbundes.

Der nicht erfindungsgemäße Anlagenverbund zur Stahlerzeugung umfasst einen Hochofen zur Roheisenerzeugung, ein Konverterstahlwerk zur Rohstahlerzeugung, ein Gasleitungssystem für Gase, die bei der Roheisenerzeugung und/oder der Rohstahlerzeugung anfallen, sowie ein Kraftwerk zur Stromerzeugung. Das Kraftwerk ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen anfallenden Hochofengichtgases und/oder eine Teilmenge des in dem Konverterstahlwerk anfallenden Konvertergases umfasst.

Im Hochofen wird aus Eisenerzen, Zuschlägen sowie Koks und anderen Reduktionsmitteln wie Kohle, Öl, Gas, Biomassen, aufbereiteten Altkunststoffen oder sonstigen Kohlenstoff und/oder Wasserstoff enthaltenden Stoffen Roheisen gewonnen. Als Produkte der Reduktionsreaktionen entstehen zwangsläufig CO, CO₂, Wasserstoff und Wasserdampf. Ein aus dem Hochofenprozess abgezogenes Hochofengichtgas weist neben den vorgenannten Bestandteilen häufig einen hohen Gehalt an Stickstoff auf. Die Gasmenge und die Zusammensetzung des Hochofengichtgases ist abhängig von den Einsatzstoffen und der Betriebsweise und unterliegt Schwankungen. Typischerweise enthält Hochofengichtgas jedoch 35 bis 60 Vol.-% N₂, 20 bis 30 Vol.-% CO, 20 bis 30 Vol.-% CO₂ und 2 bis 15 Vol.-% H₂. Rund 30 bis 40% des bei der Roheisenerzeugung entstehenden Hochofengichtgases wird im Regelfall zum Aufheizen des Heißwindes für den Hochofenprozess in Winderhitzern eingesetzt; die verbleibende Gichtgasmenge kann in anderen Werksbereichen auch extern zu Heizzwecken oder zur Stromerzeugung genutzt werden.

Im Konverterstahlwerk, das dem Hochofenprozess nachgeschaltet ist, wird Roheisen zu Rohstahl umgewandelt. Durch Aufblasen von Sauerstoff auf flüssiges Roheisen werden störende Verunreinigungen wie Kohlenstoff, Silizium, Schwefel und Phosphor entfernt. Da die Oxidationsprozesse eine starke Wärmeentwicklung verursachen, wird häufig Schrott in Mengen bis zu 25% bezogen auf das Roheisen als Kühlmittel zugesetzt. Ferner werden Kalk zur Schlackenbildung und Legierungsmittel zugegeben. Aus dem Stahlkonverter wird ein Konvertergas abgezogen, welches einen hohen Gehalt an CO aufweist und ferner Stickstoff, Wasserstoff und CO₂ enthält. Eine typische Konvertergaszusammensetzung weist 50 bis 70 Vol.-% CO, 10 bis 20 Vol.-% N₂, ca. 15 Vol.-% CO₂ und ca. 2 Vol.-% H₂ auf. Das Konvertergas wird entweder abgefackelt oder bei modernen Stahlwerken aufgefangen und einer energetischen Nutzung zugeführt.

Der nicht erfindungsgemäße Anlagenverbund kann optional im Verbund mit einer Kokerei betrieben werden. In diesem Fall umfasst der eingangs beschriebene nicht erfindungsgemäße Anlagenverbund zusätzlich eine Koksofenanlage, in der Kohle durch einen Verkokungsprozess in Koks umgewandelt wird. Bei der Verkokung von Kohle zu Koks fällt ein Koksofengas an, welches einen hohen Wasserstoffgehalt und beachtliche Mengen an CH₄ enthält. Typischerweise enthält Koksofengas 55 bis 70 Vol.-% H₂, 20 bis 30 Vol.-% CH₄, 5 bis 10 Vol.-% N₂ und 5 bis 10 Vol.-% CO. Zusätzlich weist das Koksofengas Anteile von CO₂, NH₃ und H₂S auf. In der Praxis wird das Koksofengas in verschiedenen Werksbereichen zu Heizzwecken und im Kraftwerksprozess zur Stromerzeugung genutzt. Darüber hinaus ist es bekannt, Koksofengas zusammen mit Hochofengichtgas oder mit Konvertergas zur Erzeugung von Synthesegasen zu verwenden. Gemäß einem aus WO 2010/136313 A1 bekannten Verfahren wird Koksofengas aufgetrennt in einen wasserstoffreichen Gasstrom und einen CH₄ und CO enthaltenen Restgasstrom, wobei der Restgasstrom dem Hochofenprozess zugeführt wird und der wasserstoffreiche Gasstrom mit Hochofengichtgas gemischt und zu einem Synthesegas weiterverarbeitet wird. Aus EP 0 200 880 A2 ist es bekannt, Konvertergas und Koksofengas zu mischen und als Synthesegas für eine Methanolsynthese zu nutzen. Weitere Beispiele sind aus EP2816096 A1 und HAMID GHANBARI ET AL: "Optimal design and operation of a steel plant integrated with a polygeneration system", American Institute of Chemical Engineers, AI CH E JOURNAL, Bd. 59, Nr. 10, 1. Oktober 2013 (2013-10-01), Seiten 3659-3670, bekannt.

In einem integrierten Hüttenwerk, welches im Verbund mit einer Kokerei betrieben wird, wie beispielsweise aus "Ecological Hot Metal Production Using Coke and Blast Furnance Route" (La Revue de Métallurgie, 03.2005) bekannt, werden etwa 40 bis 50 % der als Hochofengichtgas, Konvertergas und Koksofengas anfallenden Rohgase für verfahrenstechnische Prozesse eingesetzt. Etwa 50 bis 60 % der entstehenden Gase werden dem Kraftwerk zugeführt und zur Stromerzeugung genutzt. Der im Kraftwerk erzeugte Strom deckt den Strombedarf für die Roheisen- und Rohstahlerzeugung. Im Idealfall ist die Energiebilanz geschlossen, so dass abgesehen von Eisenerzen und Kohlenstoff in Form von Kohle und Koks als Energieträger kein weiterer Eintrag von Energie notwendig ist und außer Rohstahl und Schlacke kein Produkt den Anlagenverbund verlässt.

Vor diesem Hintergrund liegt der Erfindung die Aufgabe zugrunde, die Wirtschaftlichkeit des Gesamtprozesses weiter zu verbessern und ein Verfahren zum Betreiben eines Anlagenverbund anzugeben, mit dem es möglich ist, die Kosten für die Stahlerzeugung zu reduzieren.

Ausgehend von einem nicht erfindungsgemäße Anlagenverbund zur Stahlerzeugung mit einem Hochofen zur Roheisenerzeugung, einem Konverterstahlwerk zur Rohstahlerzeugung, einem Gasleitungssystem für Gase, die bei der Roheisenerzeugung und/ oder der Rohstahlerzeugung anfallen, und einem Kraftwerk zur Stromerzeugung ist eine Biotechnologieanlage vorgesehen, die an das Gasleitungssystem angeschlossen ist und hinsichtlich der Gasversorgung parallel zu dem Kraftwerk geschaltet ist. Das Gasleitungssystem umfasst eine betrieblich steuerbare Gasweiche zur Aufteilung der dem Kraftwerk und der Biotechnologieanlage zugeführten Gasmengenströme. Vorteilhafte Ausgestaltungen des Anlagenverbundes werden in den Ansprüchen 2 bis 5 beschrieben.

Gegenstand der Erfindung ist ein Verfahren nach Anspruch 1 zum Betreiben eines Anlagenverbundes, der einen Hochofen zur Roheisenerzeugung, ein Konverterstahlwerk, ein Kraftwerk und eine biotechnologische Anlage aufweist.

Gemäß dem erfindungsgemäßen Verfahren wird zumindest eine Teilmenge des bei der Roheisenerzeugung im Hochofen anfallenden Hochofengichtgases und/oder eine Teilmenge des bei der Rohstahlerzeugung anfallenden Konvertergases als Nutzgas zum Betrieb des Kraftwerkes und der biotechnologischen Anlage verwendet. Zur Deckung des Strombedarfes des Anlagenverbundes wird extern bezogener Strom und Kraftwerkstrom, der von dem Kraftwerk des Anlagenverbundes erzeugt wird, herangezogen. Dabei wird der Stromanteil des extern bezogenen Stroms bezogen auf den gesamten Strombedarf des Anlagenverbundes als variable Prozessgröße festgelegt und wird die dem Kraftwerksprozess zugeführte Nutzgasmenge in Abhängigkeit dieser Prozessgröße festgelegt. Der nicht zur Stromerzeugung genutzte Teil des Nutzgases wird nach einer Gaskonditionierung einer biotechnologischen Anlage zugeführt und für biochemische Prozesse genutzt.

In einer nicht erfindungsgemäßen Chemieanlage des Verfahrens können chemische Produkte aus Synthesegasen erzeugt werden, welche jeweils die Komponenten des Endproduktes enthalten. Chemische Produkte können beispielsweise Ammoniak oder Methanol oder auch andere Kohlenwasserstoffverbindungen sein.

Zur Herstellung von Ammoniak muss ein Synthesegas bereitgestellt werden, welches Stickstoff und Wasserstoff im richtigen Verhältnis enthält. Der Stickstoff kann aus Hochofengichtgas gewonnen werden. Als Wasserstoffquelle kann Hochofengichtgas oder Konvertergas verwendet werden, wobei Wasserstoff durch Konvertierung des CO-Anteils durch eine Wasser-Gas-Shift-Reaktion (CO + H₂O 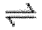 CO₂ + H₂) erzeugt wird. Zur Herstellung von Kohlenwasserstoffverbindungen, beispielsweise Methanol, muss ein im Wesentlichen aus CO und/oder CO₂ und -H₂ bestehendes Synthesegas bereitgestellt werden, welches die Komponenten Kohlenmonoxid und/oder Kohlendioxid und Wasserstoff im richtigen Verhältnis enthält. Das Verhältnis wird häufig durch das Modul (H₂ - CO₂) / (CO + CO₂) beschrieben. Der Wasserstoff kann beispielsweise durch Konvertierung des CO-Anteils im Hochofengichtgas durch eine Wasser-Gas-Shift-Reaktion erzeugt werden. Zur Bereitstellung von CO kann Konvertergas herangezogen werden. Als CO₂-Quelle kann Hochofengichtgas und/oder Konvertergas dienen.

Anstelle einer nicht erfindungsgemäßen Chemieanlage des erfindungsgemäßen Verfahrens zur Erzeugung von Produkten aus Synthesegas kann im Rahmen der Erfindung eine biotechnologische Anlage eingesetzt werden. Hierbei handelt es sich um eine Anlage zur Fermentation von Synthesegas. Das Synthesegas wird über eine Fermentation biochemisch genutzt, wobei Produkte wie Alkohole (Ethanol, Butanol), Aceton oder organische Säuren hergestellt werden können. Auch diese Produkte, die durch Fermentation von Synthesegas erzeugt werden, sind im vorliegenden Fall nur beispielhaft genannt.

Gemäß einer bevorzugten Ausführung umfasst der Anlagenverbund zusätzlich eine Koksofenanlage. Wenn die Roheisenerzeugung und die Rohstahlerzeugung im Verbund mit einer Kokerei betrieben wird, kann eine Teilmenge des bei der Roheisenerzeugung anfallenden Hochofengichtgases und/oder eine Teilmenge des im Konverterstahlwerk angefallenen Konvertergases mit einer Teilmenge des in der Koksofenanlage entstehenden Koksofengases gemischt werden und das Mischgas als Nutzgas verwendet werden. Zur Erzeugung eines Synthesegases beispielsweise für die Ammoniaksynthese kann als Rohgas eine Mischung aus Koksofengas und Hochofengichtgas oder ein Mischgas aus Koksofengas, Konvertergas und Hochofengichtgas verwendet werden. Zur Herstellung von Kohlenwasserstoffverbindungen eignet sich ein Mischgas aus Koksofengas und Konvertergas oder ein Mischgas aus Koksofengas, Konvertergas und Hochofengichtgas. Dabei sind die beschriebenen Chemieprodukte, die in einer nicht erfindungsgemäßen Chemieanlage des Verfahrens aus Hochofengichtgas, Konvertergas und Koksofengas hergestellt werden können, nur Anwendungsbeispiele zu Erläuterung der beschriebenen Verfahrensvarianten.

Die Rohgase - Koksofengas, Konvertergas und/oder Hochofengichtgas - können einzeln oder in Kombinationen als Mischgas aufbereitet und dann als Synthesegas der nicht erfindungsgemäßen Chemieanlage des erfindungsgemäßen Verfahrens zugeführt werden. Die Aufbereitung insbesondere von Kokosofengas umfasst eine Gasreinigung zur Abtrennung störender Inhaltsstoffe, insbesondere Teer, Schwefel und Schwefelverbindungen, aromatischen Kohlenwasserstoffen (BTX) und hochsiedenden Kohlenwasserstoffen. Zum Herstellen des Synthesegases ist ferner eine Gaskonditionierung notwendig. Im Rahmen der Gaskonditionierung wird der Anteil der Komponenten CO, CO₂, H₂ innerhalb des Rohgases verändert. Die Gaskonditionierung umfasst beispielsweise eine Druckwechseladsorption zur Abtrennung und Anreicherung von H₂ und/oder eine Wasser-Gas-Shift-Reaktion zur Umwandlung von CO in Wasserstoff und/oder einen Steam-Reformer zur Umwandlung des CH₄-Anteils in CO und Wasserstoff im Koksofengas.

Bei dem Verfahren wird zumindest eine Teilmenge des bei der Roheisenerzeugung im Hochofen anfallenden Hochofengichtgases und/ oder eine Teilmenge des in dem Konverterstahlwerk anfallenden Konvertergases als Rohgas nutzt, um daraus durch chemische Reaktionen in einer Chemieanlage oder erfindungsgemäß durch biochemische Prozesse in einer biotechnologischen Anlage Produkte, das heißt Wertstoffe, zu erzeugen. Gemäß einer bevorzugten Ausführung der Erfindung wird die Anlage im Verbund mit einer Kokerei betrieben und wird Koksofengas in die Nutzung einbezogen. Als Konsequenz aus der Nutzung eines Teils dieser Gase fehlt dem Anlagenverbund Strom, der extern bezogen werden muss. Der extern bezogene Strom kann aus konventionellen Kraftwerken stammen oder aus erneuerbaren Energien gewonnen werden. Vorzugsweise wird der extern bezogene Strom vollständig oder zumindest teilweise aus erneuerbarer Energie gewonnen und stammt beispielsweise aus Windkraftanlagen, Solaranlagen, geothermischen Kraftwerken, Wasserkraftwerken, Gezeitenkraftwerken und dergleichen. Zur Erreichung eines möglichst wirtschaftlichen Betriebs des Anlagenverbunds wird Strom in Zeiten niedriger Strompreise zugekauft und zur Versorgung des Anlagenverbunds verwendet und wird der nicht zur Stromerzeugung genutzte Teil des Nutzgases nach einer Gaskonditionierung erfindungsgemäß in einer biotechnologischen Anlage verwendet. In Zeiten hoher Strompreise wird das Nutzgas hingegen dem Kraftwerk vollständig oder zumindest zu einem großen Teil zugeführt, um Strom zur Versorgung des Anlagenverbundes zu erzeugen. Die biotechnologische Anlage wird in Zeiten hoher Strompreise entsprechend heruntergefahren. Zum Betrieb des Verfahrens wird eine Regelung vorgesehen, die den wechselseitigen Betrieb des Kraftwerkes einerseits und der biotechnologischen Anlage andererseits in Abhängigkeit einer variablen Prozessgröße festgelegt. Die Prozessgröße wird vorzugsweise in Abhängigkeit einer Funktion bestimmt, welche den Preis für den extern bezogenen Strom und die Kosten für die Erzeugung des Kraftwerkstroms als Variablen enthält.

Das erfindungsgemäße Verfahren ermöglicht einen wirtschaftlichen Betrieb des Anlagenverbundes. Dabei nutzt das erfindungsgemäße Verfahren insbesondere auch aus, dass der Wirkungsgrad eines Kraftwerksprozesses zur Erzeugung von Strom schlechter ist als der Wirkungsgrad einer biotechnologischen Anlage, in der durch biochemische Prozesse aus Synthesegas Chemieprodukte hergestellt werden.

Die Leistung des Kraftwerkes kann in Abhängigkeit der dem Kraftwerksprozess zugeführten Nutzgasmenge zwischen 20 % und 100 % geregelt werden. Als Kraftwerk wird vorzugsweise ein Gasturbinenkraftwerk oder ein Gas- und Dampfturbinenkraftwerk verwendet.

Die Leistung der nach der Erfindung verfahrensgemäß beanspruchten biotechnologischen Anlage wird in Abhängigkeit der dieser Anlagen zugeführten Mischgasmenge geregelt. Eine wesentliche Herausforderung für die nicht erfindungsgemäße Chemieanlage ist die dynamische Fahrweise bei wechselnden Anlagenlasten. Die Betriebsweise bei wechselnden Anlagenlasten kann insbesondere dadurch realisiert werden, dass die nicht erfindungsgemäße Chemieanlage des eine Mehrzahl parallel geschalteter kleiner Einheiten aufweist, die je nach zur Verfügung stehenden Nutzgasmengenstrom einzeln zu- oder abgeschaltet werden.

Die Verwendung einer biotechnologischen Anlage hat den Vorteil, dass eine biotechnologische Anlage flexibler hinsichtlich Lastwechsel ist als eine Chemieanlage.

Nicht unter die Erfindung fällt ferner die Verwendung einer Chemieanlage zur Ankopplung an ein Hüttenwerk nach Anspruch 17 und die Verwendung einer biotechnologischen Anlage zur Ankopplung an ein Hüttenwerk entsprechend Anspruch 18.

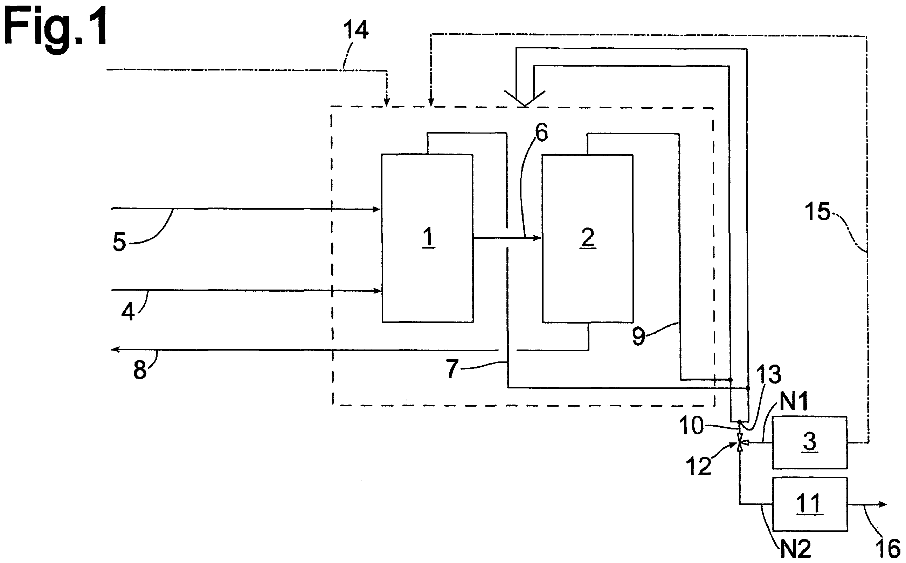

Im Folgenden wird die Erfindung anhand einer lediglich ein Ausführungsbeispiel darstellenden Zeichnung erläutert. Es zeigen schematisch
- Fig. 1: ein stark vereinfachtes Blockschaltbild eines nicht erfindungsgemäßen Anlagenverbundes zur Stahlerzeugung mit einem Hochofen zur Roheisenerzeugung und einem Konverterstahlwerk zur Rohstahlerzeugung, einem Kraftwerk und einer erfindungsgemäßen Biotechnologieanlage oder nach der nicht erfindungsgemäßen Chemieanlage,

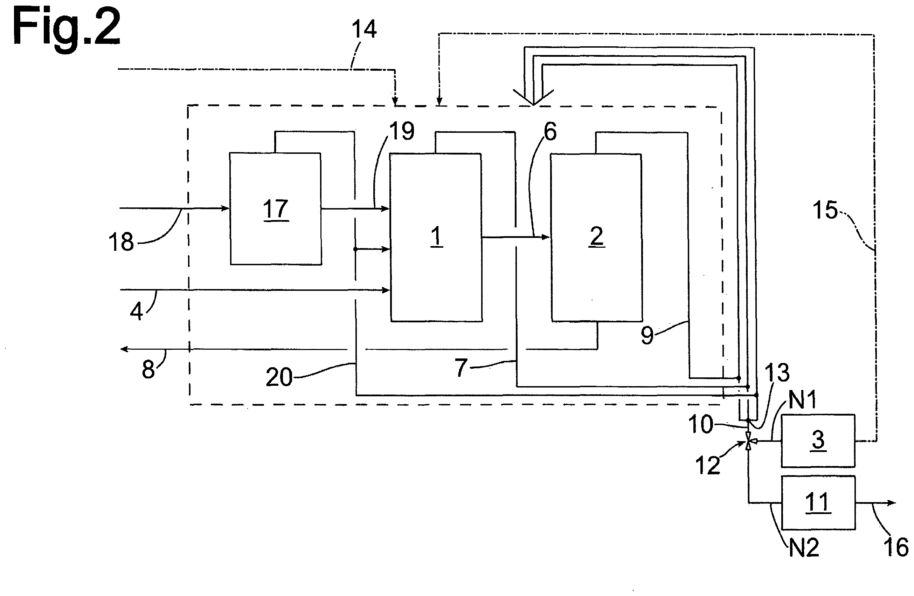

- Fig. 2: das stark vereinfachte Blockschaltbild eines nicht erfindungsgemäßen Anlagenverbundes, der zusätzlich zu einem Hochofen zur Roheisenerzeugung und einem Konverterstahlwerk zur Rohstahlerzeugung einem Kraftwerk und einer nicht erfindungsgemäßen Chemie- oder erfindungsgemäßen Biotechnologieanlage auch eine Koksofenanlage umfasst,
- Fig.: 3 das Blockschaltbild eines nicht erfindungsgemäßen Anlagenverbundes entsprechend Fig. 2 mit einer zusätzlichen Anlage zur Wasserstofferzeugung.

Der in Fig. 1 dargestellte Anlagenverbund zur Stahlerzeugung umfasst einen Hochofen 1 zur Roheisenerzeugung, ein Konverterstahlwerk 2 zur Rohstahlerzeugung, ein Kraftwerk 3 zur Stromerzeugung und eine erfindungsgemäße Biotechnologieanlage 11 oder eine nicht erfindungsgemäße Chemieanlage .

Im Hochofen 1 wird im Wesentlichen aus Eisenerz 4 und Reduktionsmitteln 5, insbesondere Koks und Kohle, Roheisen 6 gewonnen. Durch Reduktionsreaktionen entsteht ein Hochofengichtgas 7, welches als Hauptbestandteile Stickstoff, CO, CO₂ und H₂ enthält. Im Konverterstahlwerk 2, das dem Hochofenprozess nachgeschaltet ist, wird Roheisen 6 zu Rohstahl 8 umgewandelt. Durch Aufblasen von Sauerstoff auf das flüssige Roheisen werden störende Verunreinigungen, insbesondere Kohlenstoff, Silizium und Phosphor entfernt. Zur Kühlung kann Schrott in Mengen bis zu 25 % bezogen auf die Roheisenmenge zugeführt werden. Ferner werden Kalk zur Schlackenbildung und Legierungsmittel zugegeben. Am Kopf des Konverters wird ein Konvertergas 9 abgezogen, welches einen sehr hohen Anteil an CO aufweist.

Das Kraftwerk 3 ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen 1 anfallenden Hochofengichtgases 7 und eine Teilmenge des im Konverterstahlwerk 2 anfallenden Konvertergases 9 umfasst. Zur Führung der Gase ist ein Gasleitungssystem vorgesehen.

Gemäß einer in Fig. 1 dargestellten Gesamtbilanz wird dem Anlagenverbund Kohlenstoff als Reduktionsmittel 5 in Form von Kohle und Koks sowie Eisenerz 4 zugeführt. Als Produkte fallen Rohstahl 8 und Rohgase 7, 9 an, die sich in Menge, Zusammensetzung, Heizwert und Reinheit unterscheiden und an verschiedenen Stellen im Anlagenverbund wieder eingesetzt werden. Bei einer Gesamtbetrachtung wird 40 bis 50 %, zumeist etwa 45 %, der Rohgase 7, 9 wieder in den Hüttenprozess zur Roheisenerzeugung oder Rohstahlerzeugung zurückgeführt. Zwischen 50 und 60 %, zumeist etwa 55 %, der Rohgase 7, 9 kann zum Betrieb des Kraftwerkes 3 genutzt werden. Das mit einem Mischgas 10 aus Hochofengichtgas 7 und Konvertergas 9 betriebene Kraftwerk 3 wird so ausgelegt, dass es den Strombedarf des Anlagenverbundes decken kann.

Gemäß der Darstellung in Fig. 1 ist eine erfindungsgemäße Biotechnologieanlage 11 oder eine nicht erfindungsgemäße Chemieanlage vorgesehen, die an das Gasleitungssystem angeschlossen ist und hinsichtlich der Gasversorgung parallel zu dem Kraftwerk 3 geschaltet ist. Das Gasleitungssystem weist eine betrieblich steuerbare Gasweiche 12 zur Aufteilung der dem Kraftwerk 3 und der nicht erfindungsgemäßen Chemie- oder erfindungsgemäßen Biotechnologieanlage 11 zugeführten Gasmengeströme auf. In Strömungsrichtung vor der Gasweiche ist eine Mischvorrichtung 13 zur Herstellung des aus Hochofengichtgas 7 und Konvertergas 9 bestehenden Mischgases 10 vorgesehen.

Bei dem in Fig. 1 dargestellten nicht erfindungsgemäßen Anlagenverbund wird zumindest eine Teilmenge des bei der Roheisenerzeugung im Hochofen 1 anfallenden Hochofengichtgases 7 und eine Teilmenge des bei der Rohstahlerzeugung anfallenden Konvertergases 9 als Nutzgas zum Betrieb des Kraftwerkes 3 und der Biotechnologieanlage 11 oder der nicht erfindungsgemäßen Chemieanlage verwendet. Zur Deckung des Strombedarfes des Anlageverbundes wird extern bezogener Strom 14 und Kraftwerkstrom 15, der von dem Kraftwerk 3 des Anlagenverbundes erzeugt wird, herangezogen. Der Stromanteil des extern bezogenen Stroms 14 bezogen auf den gesamten Strombedarf des Anlagenverbundes wird als variable Prozessgröße festgelegt und die dem Kraftwerk 3 zugeführte Nutzgasmenge N1 wird in Abhängigkeit dieser Prozessgröße bestimmt. Der nicht zur Stromerzeugung genutzte Teil des Nutzgases N2 wird nach einer Gaskonditionierung als Synthesegas zur Herstellung chemischer Produkte 16 verwendet oder nach einer Gaskonditionierung der biotechnologischen Anlage zugeführt und für biochemische Prozesse genutzt.

Der extern bezogene Strom 14 wird vorzugsweise vollständig oder zumindest teilweise aus erneuerbarer Energie gewonnen und stammt beispielsweise aus Windkraftanlagen, Solaranlagen, Wasserkraftwerken und dergleichen. Die Prozessgröße, auf deren Grundlage die dem Kraftwerksprozess zugeführte Nutzgasmenge N1 festgelegt wird, wird in Abhängigkeit einer Funktion bestimmt, welche den Preis für den extern bezogenen Strom und die Kosten für die Erzeugung des Kraftwerkstromes 15 als Variablen enthält. Zur Erreichung eines möglichst wirtschaftlichen Betriebs des Anlagenverbundes wird Strom in Zeiten niedriger Strompreise als externer Strom 14 zugekauft und zur Stromversorgung des Anlagenverbundes verwendet, wobei der nicht zur Stromerzeugung genutzte Teil des Nutzgases N2 der Biotechnologieanlage 11 oder der nicht erfindungsgemäßen Chemieanlage zugeführt und nach einer Gaskonditionierung als Synthesegas zur Herstellung chemischer Produkte 16 verwendet wird. In Zeiten hoher Strompreise werden die bei der Roheisenerzeugung und Rohstahlerzeugung anfallenden Rohgase 7, 9 dem Kraftwerk 3 zugeführt, um den Strom zur Versorgung des Anlagenverbundes zu erzeugen. Die biotechnologische Anlage oder die alternativ vorgesehene nicht erfindungsgemäße Chemieanlage 11 wird in Zeiten hoher Strompreise entsprechend heruntergefahren.

Die Leistung des Kraftwerkes 3 wird in Abhängigkeit der dem Kraftwerkprozess zugeführten Nutzgasmenge N1 zwischen 20 % und 100 % geregelt. Die Leistung der biotechnologischen Anlage oder der nicht erfindungsgemäßen Chemieanlage 11 wird in Abhängigkeit der dieser Anlage zugeführten Nutzgasmenge N2 geregelt. Eine wesentliche Herausforderung für die nicht erfindungsgemäße Chemieanlage 11 ist die dynamische Betriebsweise bei wechselnden Lasten. Diese kann dadurch realisiert werden, dass die nicht erfindungsgemäße Chemieanlage 11 eine Mehrzahl parallel geschalteter kleiner Einheiten aufweist, die je nach zur Verfügung stehender Nutzgasmenge N2 einzeln zu- oder abgeschaltet werden.

Im Ausführungsbeispiel der Fig. 2 umfasst der nicht erfindungsgemäße Anlagenverbund zusätzlich eine Koksofenanlage 17. Bei der Verkokung von Kohle 18 zu Koks 19 fällt Koksofengas 20 an, welches einen hohen Anteil an Wasserstoff und CH₄ enthält. Teile des Koksofengases 20 können für die Beheizung der Winderhitzer im Hochofen 1 genutzt werden. Das Gasleitungssystem schließt eine Gasverteilung für das Koksofengas 20 ein. In Strömungsrichtung vor der Gasweiche 12 ist eine Mischvorrichtung 13 zur Herstellung eines aus Hochofengichtgas 7, Konvertergas 9 und Koksofengas 20 bestehenden Mischgases 10 vorgesehen. Mit der Gasweiche sind die dem Kraftwerk 3 und der Biotechnologieanlage 11 oder der nach der nicht erfindungsgemäßen Chemieanlage zugeführten Gasmengenströme steuerbar.

Beim Betrieb der in Fig. 2 dargestellten Anlage wird eine Teilmenge des bei der Roheisenerzeugung anfallenden Hochofengichtgases 7 und/oder eine Teilmenge des im Konverterstahlwerk anfallenden Konvertergases 9 mit einer Teilmenge des in der Koksofenanlage 17 entstehenden Koksofengases 20 gemischt. Das Mischgas 10 wird als Nutzgas zum Betrieb des Kraftwerkes 3 und der biotechnologischen Anlage oder nach der nicht erfindungsgemäßen Chemieanlage 11 verwendet.

Das Hochofengichtgas 7, das Konvertergas 9 und das Koksofengas 20 können beliebig miteinander kombiniert werden. Die Kombination der Gasströme 7, 9, 20 richtet sich nach dem gewünschten Synthesegas bzw. dem Produkt, welches in der der biotechnologischen Anlage oder der nach der nicht erfindungsgemäßen Chemieanlage 11 unter Verwendung des Synthesegases hergestellt werden soll.

Im Rahmen der Erfindung ist es beispielsweise möglich, dass Hochofengichtgas 7 und Konvertergas 9 gemischt werden, dass aus dem Mischgas nach einer Gaskonditionierung ein Synthesegas hergestellt wird und dass dem Synthesegas oder dem gereinigten Mischgas vor der Weiterverarbeitung zu dem Synthesegas zusätzlich aufbereitetes Koksofengas 20 zugemischt wird.

Ferner besteht die Möglichkeit, dass aus Hochofengichtgas 7 nach einer Gaskonditionierung ein Synthesegas hergestellt wird und dass dem Synthesegas oder dem gereinigten Hochofengichtgas vor der Weiterverarbeitung zu dem Synthesegas zusätzlich aufbereitetes Koksofengas 20 zugemischt wird.

Schließlich besteht die Möglichkeit, dass aus Konvertergas 9 nach einer Gaskonditionierung ein Synthesegas hergestellt wird und dass dem Synthesegas oder dem gereinigten Konvertergas vor der Weiterverarbeitung zu dem Synthesegas zusätzlich aufbereitetes Koksofengas 20 zugemischt wird.

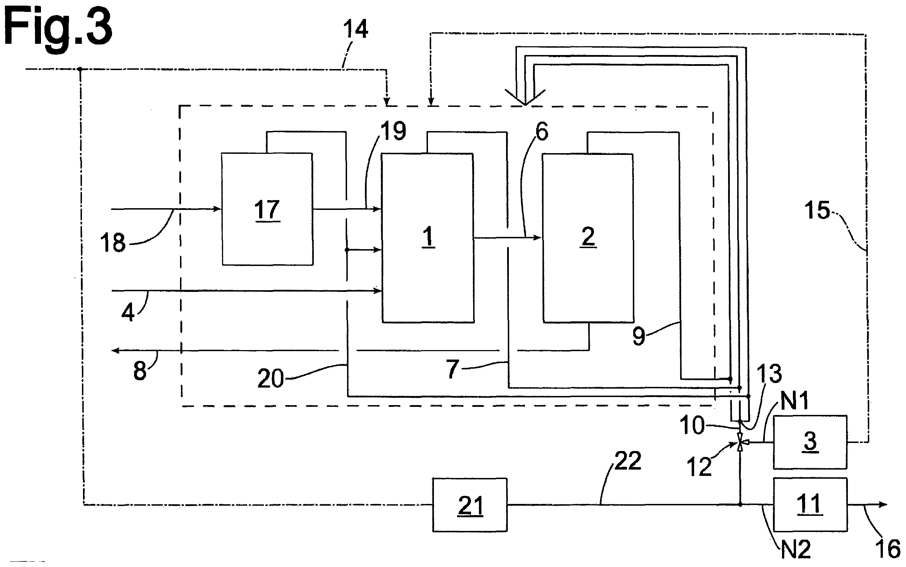

Bei den in den Fig. 1 und 2 dargestellten Betriebsweise kann der Kohlenstoffgehalt und der Stickstoffgehalt der beim Betrieb des Anlageverbundes anfallenden Rohgase nicht vollständig zur Herstellung von chemischen Produkten genutzt werden, da ein Wasserstoffunterschuss vorliegt. Um den Kohlenstoffgehalt und den Stickstoffgehalt des Nutzgases vollständig für die Herstellung von chemischen Wertstoffen einzusetzen, weist der in Fig. 3 dargestellte nicht erfindungsgemäße Anlagenverbund zusätzlich eine Anlage 21 zur Wasserstofferzeugung auf, die durch eine wasserstoffführende Leitung 22 mit dem Gasleitungssystem verbunden ist. Die Anlage 21 zur Wasserstofferzeugung kann insbesondere eine Elektrolyseanlage zur Wasserelektrolyse sein. Der Betrieb einer Wasserelektrolyse ist energieintensiv und wird daher primär in Zeiten niedriger Strompreise in Betrieb genommen, zu denen auch die Biotechnologieanlage oder die nach der nicht erfindungsgemäßen Chemieanlage 11 betrieben wird und das Kraftwerk 3 heruntergefahren ist. Der zusätzlich erzeugte Wasserstoff wird der nach der nicht erfindungsgemäßen Chemieanlage 11 zusammen mit dem Mischgas zugeführt. Dadurch kann die Kapazität der nach der nicht erfindungsgemäßen Chemieanlage 11 deutlich gesteigert werden. Entsprechendes gilt, wenn anstelle der nach der nicht erfindungsgemäßen Chemieanlage 11 eine erfindungsgemäße biotechnologische Anlage vorgesehen wird.

## Patentansprüche

1. Verfahren zum Betreiben eines Anlagenverbundes, der einen Hochofen (1) zur Roheisenerzeugung, ein Konverterstahlwerk (2), ein Kraftwerk (3) und eine biotechnologische Anlage (11) aufweist,
a) wobei zumindest eine Teilmenge des bei der Roheisenerzeugung im Hochofen (1) anfallenden Hochofengichtgases (7) und/oder eine Teilmenge des bei der Rohstahlerzeugung anfallenden Konvertergases (9) als Nutzgas zum Betrieb des Kraftwerkes (3) und der biotechnologischen Anlage (11) verwendet wird,
b) wobei zur Deckung des Strombedarfes des Anlagenverbundes extern bezogener Strom (14) und Kraftwerkstrom (15), der von dem Kraftwerk (3) des Anlagenverbundes erzeugt wird, herangezogen wird,
c) wobei der Stromanteil des extern bezogenen Stroms (14) bezogen auf den gesamten Strombedarf des Anlagenverbundes als variable Prozessgröße festgelegt wird und die dem Kraftwerksprozess zugeführte Nutzgasmenge (N1) in Abhängigkeit dieser Prozessgröße festgelegt wird,
d) wobei der nicht zur Stromerzeugung genutzte Teil des Nutzgases (N2) nach einer Gaskonditionierung der biotechnologischen Anlage zugeführt und für biochemische Fermentations-Prozesse genutzt wird.

2. Verfahren nach Anspruch 1, **dadurch gekennzeichnet, dass** der Anlagenverbund zusätzlich eine Koksofenanlage (17) umfasst und dass eine Teilmenge des bei der Roheisenerzeugung anfallenden Hochofengichtgases (7) und/oder eine Teilmenge des im Konverterstahlwerk (2) anfallenden Konvertergases (9) mit einer Teilmenge des in der Koksofenanlage (17) entstehenden Koksofengases (20) gemischt wird und dass das Mischgas (10) als Nutzgas verwendet wird.

3. Verfahren nach Anspruch 2, **dadurch gekennzeichnet, dass** Hochofengichtgas (7) und Konvertergas (9) gemischt werden, dass aus dem Mischgas (10) nach einer Gaskonditionierung ein Synthesegas hergestellt wird und dass dem Synthesegas oder dem gereinigten Mischgas (11) vor der Weiterverarbeitung zu dem Synthesegas zusätzlich aufbereitetes Koksofengas (20) zugemischt wird.

4. Verfahren nach Anspruch 2, **dadurch gekennzeichnet, dass** aus Hochofengichtgas (7) nach einer Gaskonditionierung ein Synthesegas hergestellt wird und dass dem Synthesegas oder dem gereinigten Hochofengichtgas vor der Weiterverarbeitung zu dem Synthesegas zusätzlich aufbereitetes Koksofengas (20) zugemischt wird.

5. Verfahren nach Anspruch 2, **dadurch gekennzeichnet, dass** aus Konvertergas (9) nach einer Gaskonditionierung ein Synthesegas hergestellt wird und dass dem Synthesegas oder dem gereinigten Konvertergas vor der Weiterverarbeitung zu dem Synthesegas zusätzlich aufbereitetes Koksofengas (20) zugemischt wird.

6. Verfahren nach einem der Ansprüche 1 bis 5, **dadurch gekennzeichnet, dass** der extern bezogene Strom (14) vollständig oder zumindest teilweise aus erneuerbarer Energie gewonnen wird.

7. Verfahren nach einem der Ansprüche 1 bis 6, **dadurch gekennzeichnet, dass** die Prozessgröße in Abhängigkeit einer Funktion bestimmt wird, welche den Preis für den extern bezogenen Strom (14) und die Kosten für die Erzeugung des Kraftwerkstromes (15) als Variablen enthält.

8. Verfahren nach Anspruch 7, **dadurch gekennzeichnet, dass** die Leistung des Kraftwerkes (3) in Abhängigkeit der dem Kraftwerksprozess zugeführten Nutzgasmenge (N1) zwischen 20 % und 100 % geregelt wird.

9. Verfahren nach einem der Ansprüche 1 bis 8, **dadurch gekennzeichnet, dass** als Kraftwerk (3) ein Gasturbinenkraftwerk oder ein Gas- und Dampfturbinenkraftwerk verwendet wird.

10. Verfahren nach einem der Ansprüche 1 bis 9, **dadurch gekennzeichnet, dass** die Leistung der biotechnologischen Anlage in Abhängigkeit der dieser Anlage zugeführten Mischgasmenge (N2) geregelt wird.

## Claims

1. Method for operating a plant complex which comprises a blast furnace (1) for producing pig iron, a converter steel mill (2), a power-generating plant (3) and a biotechnological plant (11),
a) wherein at least a partial amount of the blast-furnace top gas (7) occurring in the production of pig iron in the blast furnace (1) and/or a partial amount of the converter gas (9) occurring in the production of crude steel is used as a useful gas for operating the power-generating plant (3) and the biotechnological plant (11),
b) wherein externally obtained electricity (14) and power-generating plant electricity (15), which is produced by the power-generating plant (3) of the plant complex, is used to cover the electricity demand of the plant complex,
c) wherein the proportion of electricity accounted for by the externally obtained electricity (14) with respect to the overall electricity demand of the plant complex is established as a variable process parameter and the amount of useful gas (N1) fed to the power-generating process is established in dependence on this process parameter,
d) wherein the part of the useful gas (N2) that is not used for electricity generation is fed to the biotechnological plant after a gas-conditioning operation and is used for biochemical fermentation processes.

2. Method according to Claim 1, **characterized in that** the plant complex additionally comprises a coke-oven plant (17), and **in that** a partial amount of the blast-furnace top gas (7) occurring in the production of pig iron and/or a partial amount of the converter gas (9) occurring in the converter steel mill (2) is mixed with a partial amount of the coke-oven gas (20) occurring in the coke-oven plant (17), and **in that** the mixed gas (10) is used as a useful gas.

3. Method according to Claim 2, **characterized in that** blast-furnace top gas (7) and converter gas (9) are mixed, **in that** a syngas is produced from the mixed gas (10) after a gas-conditioning operation, and **in that** conditioned coke-oven gas (20) is additionally admixed with the syngas or the cleaned mixed gas (11) before the further processing to form the syngas.

4. Method according to Claim 2, **characterized in that** a syngas is produced from blast-furnace top gas (7) after a gas-conditioning operation, and **in that** conditioned coke-oven gas (20) is additionally admixed with the syngas or the cleaned blast-furnace top gas before the further processing to form the syngas.

5. Method according to Claim 2, **characterized in that** a syngas is produced from converter gas (9) after a gas-conditioning operation, and **in that** conditioned coke-oven gas (20) is additionally admixed with the syngas or the cleaned converter gas before the further processing to form the syngas.

6. Method according to one of Claims 1 to 5, **characterized in that** the externally obtained electricity (14) is completely or at least partially obtained from renewable energy.

7. Method according to one of Claims 1 to 6, **characterized in that** the process parameter is determined in dependence on a function that includes the price for the externally obtained electricity (14) and the costs for producing the power-generating plant electricity (15) as variables.

8. Method according to Claim 7, **characterized in that** the power output of the power-generating plant (3) is controlled between 20*%* and 100%, in dependence on the amount of useful gas (N1) fed to the power-generating process.

9. Method according to one of Claims 1 to 8, **characterized in that** a gas-turbine power-generating plant or a gas-turbine and steam-turbine power-generating plant is used as the power-generating plant (3).

10. Method according to one of Claims 1 to 9, **characterized in that** the power output of the biotechnological plant is controlled in dependence on the amount of mixed gas (N2) fed to this plant.

## Revendications

1. Procédé d'exploitation d'un ensemble d'installations qui comprend un haut fourneau (1) pour la production de fonte brute, une aciérie à convertisseur (2), une centrale électrique (3) et une installation biotechnologique (11),
a) au moins une quantité partielle du gaz de haut fourneau (7) formé lors de la production de fonte brute dans le haut fourneau (1) et/ou une quantité partielle du gaz de convertisseur (9) formé lors de la production d'acier brut étant utilisées en tant que gaz utile pour l'exploitation de la centrale électrique (3) et de l'installation biotechnologique (11),
b) de l'électricité (14) achetée en externe et de l'électricité de centrale électrique (15) qui est produite par la centrale électrique (3) de l'ensemble d'installations étant utilisées pour couvrir le besoin en électricité de l'ensemble d'installations,
c) la proportion d'électricité de l'électricité achetée en externe (14) par rapport à la totalité du besoin en électricité de l'ensemble d'installations étant définie en tant que grandeur de processus variable et la quantité de gaz utile (N1) amenée au processus de la centrale électrique étant définie en fonction de cette grandeur de processus,
d) la partie du gaz utile (N2) non utilisée pour la production d'électricité étant amenée à l'installation biotechnologique après un conditionnement de gaz et utilisée pour des processus de fermentation biochimiques.

2. Procédé selon la revendication 1, **caractérisé en ce que** l'ensemble d'installations comprend en outre une installation de four à coke (17) et **en ce qu'**une quantité partielle du gaz de haut fourneau (7) formé lors de la production de fonte brute et/ou une quantité partielle du gaz de convertisseur (9) formé dans l'aciérie à convertisseur (2) sont mélangées avec une quantité partielle du gaz de four à coke (20) formé dans l'installation de four à coke (17) et **en ce que** le gaz mélangé (10) est utilisé en tant que gaz utile.

3. Procédé selon la revendication 2, **caractérisé en ce que** le gaz de haut fourneau (7) et le gaz de convertisseur (9) sont mélangés, **en ce qu'**un gaz de synthèse est produit à partir du gaz mélangé (10) après un conditionnement de gaz et **en ce que** du gaz de four à coke (20) préparé en outre est mélangé au gaz de synthèse ou au gaz mélangé purifié (11) avant la transformation ultérieure en le gaz de synthèse.

4. Procédé selon la revendication 2, **caractérisé en ce qu'un** gaz de synthèse est produit à partir de gaz de haut fourneau (7) après un conditionnement de gaz et **en ce que** du gaz de four à coke (20) préparé en outre est mélangé au gaz de synthèse ou au gaz de haut fourneau purifié avant la transformation ultérieure en le gaz de synthèse.

5. Procédé selon la revendication 2, **caractérisé en ce qu**'un gaz de synthèse est produit à partir du gaz de convertisseur (9) après un conditionnement de gaz et en ce que du gaz de four à coke (20) préparé en outre est mélangé au gaz de synthèse ou au gaz de convertisseur purifié avant la transformation ultérieure en le gaz de synthèse.

6. Procédé selon l'une quelconque des revendications 1 à 5, **caractérisé en ce que** l'électricité (14) achetée en externe est entièrement ou au moins partiellement obtenue à partir d'énergie renouvelable.

7. Procédé selon l'une quelconque des revendications 1 à 6, **caractérisé en ce que** la grandeur de processus est déterminée en fonction d'une fonction qui contient en tant que variables le prix de l'électricité achetée en externe (14) et le coût de la production de l'électricité de centrale électrique (15).

8. Procédé selon la revendication 7, **caractérisé en ce que** la puissance de la centrale électrique (3) est réglée entre 20 % et 100 % en fonction de la quantité de gaz utile (N1) amenée au processus de la centrale électrique.

9. Procédé selon l'une quelconque des revendications 1 à 8, **caractérisé en ce qu**'une centrale électrique à turbine à gaz ou une centrale électrique à turbine à gaz et à vapeur est utilisée en tant que centrale électrique (3) .

10. Procédé selon l'une quelconque des revendications 1 à 9, **caractérisé en ce que** la puissance de l'installation biotechnologique est réglée en fonction de la quantité de gaz mélangé (N2) amenée à cette installation.
